# EUROPÄISCHE PATENTSCHRIFT

(11) **EP 0 508 220 B1**
(45) Veröffentlichungstag und Bekanntmachung des Hinweises auf die Patenterteilung: **26.07.1995**
(21) Anmeldenummer: 92105138.9
(22) Anmeldetag: 25.03.1992
(51) Int. Cl.: C07K 5/06, A61K 38/05, C07C 257/18

(54) **Amidinophenylalaninderivate, Verfahren zu deren Herstellung, deren Verwendung und diese enthaltende Mittel**
Derivatives of amindinophenylalanine, procedure for their preparation, their utilisation and compositions comprising them
Dérivées d'amidino-phenylalanine, procédé de leur préparation, leur utilisation et composition les contenants

(30) Priorität: 09.04.1991 DE 4111394
(43) Veröffentlichungstag der Anmeldung: 14.10.1992
(73) Patentinhaber: BEHRINGWERKE Aktiengesellschaft, 35001 Marburg (DE)
(72) Erfinder: Stüber, Werner, W-3551 Lahntal 3 (DE); Dickneite, Gerhard, W-3550 Marburg (DE)

(56) Entgegenhaltungen:
- EP-A- 0 236 163
- EP-A- 0 236 164
- CHEMICAL ABSTRACTS, Band 110, Nr. 11, 13. MUrz 1989, Columbus, Ohio, USA B. VOIGT et al. "Synthesis of N Alpha-(arylsulfonyl)-4- -amidinophenyl-
- alanylprolines and N Alpha-(arylsulfonyl- glycyl)-4-amidinophenyl- - alanylprolines and their testing as inhibitors of serine proteases. Part 33: Synthetic inhibitors of serine proteases." Seite 760, Spalte 2, Zusammenfassung-Nr. 95 774g
- CHEMICAL ABSTRACTS, Band 110, Nr. 7, 03. Februar 1989, Columbus, Ohio, USA H.P. KLOECKING et al. "Influence of Alpha-NAPAP in thrombininduced release of plasminogen activator" Seite 38, Spalte 2, Zusammenfassung-Nr. 50 941t
- CHEMICAL ABSTRACTS, Band 111, Nr. 9, 28. August 1989, Columbus, Ohio, USA J. HAUPTMANN et al. "Pharma- cological characterization of a new structural variant of a 4-amidinophenylalanine amide-type synthetic thrombin inhibitor" Seite 46, Spalte 1, Zusammenfassung-Nr. 70 596h

## Beschreibung

Die Erfindung betrifft Amidinophenylalaninderivate, Verfahren zu ihrer Herstellung, ihre Verwendung sowie pharmazeutische Mittel, die diese Verbindungen enthalten.

Bekanntlich führen eine Reihe von pathophysiologischen Bedingungen zu einem Verbrauch von Antithrombin III (AT III), dem wichtigsten Thrombininhibitor im Plasma. Ein Abfall von AT III führt zu einem erhöhten Thromboserisiko, wie man u. a. auch von Fällen mit angeborenem Mangel an AT III weiß. Ein Abfall auf Werte unterhalb 75 % der Norm hat thromboembolische Komplikationen zur Folge. Diese Komplikationen treten häufig in Form einer disseminierten intravasalen Gerinnung nach Operationen und bei Schockzuständen auf. In vielen Fällen treten dabei lebensbedrohliche Blutgerinnsel auf. Zur Therapie und Prophylaxe thrombotischer Erkrankungen werden bisher in der Medizin Antikoagulantien mit unterschiedlicher Wirkungsweise eingesetzt. Zur akuten Bekämpfung eines Thromboserisikos werden Substanzen wie AT III, Heparin und neuerdings auch Hirudin eingesetzt. Langzeitprophylaxen werden mit Cumarin- und Indandionderivaten ausgeführt. Die aufgeführten Antikoagulantien sind jedoch mit z. T. erheblichen Nachteilen behaftet.

Heparin beispielsweise kann aufgrund seiner Polysaccharidstruktur nur parenteral appliziert werden und seine Wirkung ist auch auf einen funktionsfähigen Antithrombin III-Spiegel angewiesen. Cumarine wirken direkt der Proteinbiosynthese entgegen, indem die Vitamin K-abhängigen Gerinnungsfaktoren II, VII, IX und X nicht mehr ausreichend zur Verfügung gestellt werden und somit das Gerinnungspotential reduziert wird. Daraus ergibt sich eine zeitliche Verzögerung der Wirkung. Als bekannte Nebenwirkungen gelten hämorrhagische Hautnekrosen, Nausea und Haarausfall.

Demgegenüber haben niedermolekulare Thrombininhibitoren den Vorteil, daß sie kofaktorenunabhängig direkt auf Thrombin einwirken, indem sie direkt an das aktive Zentrum binden und damit das Enzym gleichsam verschliessen. Aufgrund ihrer chemischen Struktur können diese Substanzen oral appliziert werden und entfalten ihre Wirkung unverzüglich.

Besondere Bekanntheit haben Aminosäurederivate auf Argininbasis oder Amidinophenylalaninbasis erlangt. Zur ersten Gruppe zählen Verbindungen wie D-Phenylalanyl-L-prolyl-argininaldehyd und (2R,4R)-4-Methyl-1-[N2-(3-methyl-1,2,3,4-tetrahydro-8-chinolinsulfonyl-L-arginyl]-2-piperidincarbonsäure monohydrat ("MD 805"). MD 805 ist ein kompetitiver spezifischer Thrombininhibitor, der auch therapeutisch eingesetzt wird. Ein weiteres bekanntes Amidinophenylalaninderivat ist das betaNaphthylsulfonyl-glycyl-R,S-4-amidinophenylalanylpiperidid (NAPAP). In EP 0 236 163 und EP 0 236 164 sind Derivate des NAPAPs beschrieben. In diesen ist Glycin durch eine Aminosäure der Struktur NH2-CHR1-COOH, worin R1 eine niedere Alkylgruppe, eine niedere Hydroxyalkylgruppe, eine Phenylgruppe oder eine 4-Hydroxyphenylgruppe ist, ersetzt. Das 4-Amidinophenylalanin (Aph) kann zu N-methyl-Aph N-methyliert sein. Außerdem wurden für NAPAP verschiedene Derivatisierungen am Arylsulfonyl, "Brücken"-Glycin und am Piperidinring beschrieben. Am geeignetsten sind demnach alpha- oder beta-Naphthylsulfonylgruppen am N-Terminus, wogegen Heteroarylsulfonylgruppen wie 8-Chinolinsulfonyl um Zehnerpotenzen schlechter sind. Ein Nachteil von natürlichen Aminosäuren als Brückenglied zwischen dem hydrophoben Naphthylrest und Aph ist die enzymatische Spaltbarkeit der Amidbindung zum Aph. Besonders wird dieser Nachteil bei einer oralen Verabreichung zum Tragen kommen. Ein Ersatz durch andere Brückenglieder wie beta Alanin anstelle Glycin führt jedoch zu deutlichen Wirksamkeitsverlusten bezüglich der Thrombininhibition. Auch ein Ersatz durch Iminosäuren wie Prolin führt zu einem Wirkungsverlust.

Ziel der Erfindung war es deshalb, neue Verbindungen auf Basis von Amidinophenylalanin bereitzustellen, die bekannten Verbindungen in ihrer antithrombotischen Wirksamkeit überlegen sind und eine hohe enzymatische Resistenz bei gleichzeitig verbesserter Verträglichkeit aufweisen.

Gegenstand dieser Erfindung sind demnach Verbindungen der Formel I: 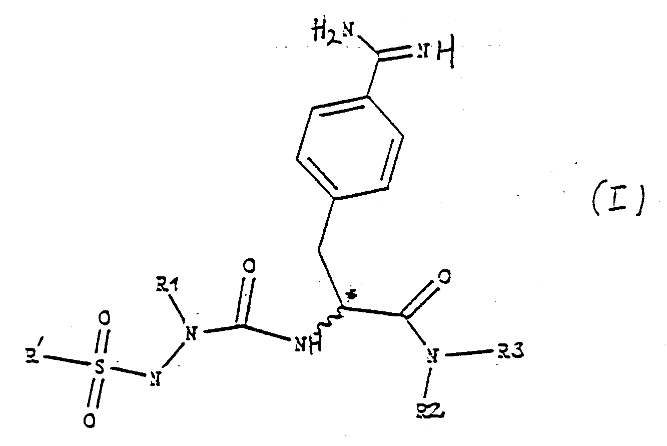
worin
- R': eine Naphthyl- oder Phenylgruppe, die mit ein bis drei Alkoxygruppen, enthaltend bis zu drei C-Atome, oder mit bis zu fünf Alkylgruppen, enthaltend ein bis zwei C-Atome, derivatisiert sein kann, oder eine Chromangruppe, die mit bis zu fünf Methylgruppen derivatisiert sein kann,
- R1: Wasserstoff, eine niedere Alkylgruppe mit bis zu 4 C-Atomen, eine Hydroxyalkylgruppe, eine Aralkylgruppe mit bis zu 7 Kohlenstoffatomen oder eine Carboxyalkylgruppe mit bis zu 4 C-Atomen,
- R2 und R3: gleich oder verschieden je eine Alkylgruppe mit bis zu 4 C-Atomen bedeuten, wobei R² und R³ mit dem Stickstoffatom einen Ring bilden können, der mit einer Carboxylgruppe, Hydroxylgruppe oder Hydroxyalkylgruppe mit bis zu 3 Kohlenstoffatomen derivatisiert sein kann, und
- *: in der R oder S Struktur, vorzugsweise aber in der R-Struktur vorliegt.

Ein wesentlicher Unterschied zu den bekannten Strukturen besteht darin, daß das Atom, an das R¹ gebunden ist, ein Stickstoffatom statt ein Kohlenstoffatom ist.

NAPAP hat folgende Struktur:

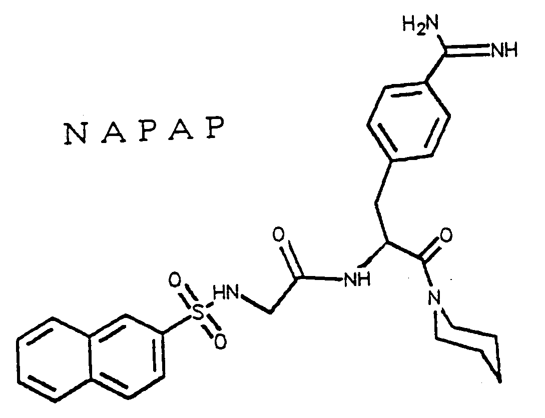

Überraschenderweise konnte die antithrombotische Aktivität durch einen Ersatz des Glycins in NAPAP durch Azaaminosäurereste erheblich gesteigert werden. Azaaminosäurereste sind bekannte Verbindungen, die sich durch die folgende Formel darstellen lassen:

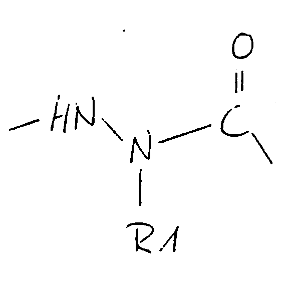

Das Strukturelement der Azaaminosäuren führte zu Strukturen wie oben in I dargestellt.

Überraschenderweise übertrifft β-Naphthyl-sulfonylazaglycyl-D-amidinophenylalanyl-piperidid die Wirksamkeit der Glycinverbindung um den 5-fachen Wert. Die Kombination von Azaaminosäuren mit hydrophoben Resten R' gemäß Formel I führt zu Verbindungen mit Ki- und IC50-Werten im picomolaren Bereich. Darüber hinaus zeigen die Azaaminosäuren enthaltenden Verbindungen eine Resistenz gegenüber enzymatischem Abbau, so daß die erfindungsgemäßen Verbindungen neben einer beträchtlichen Wirkungssteigerung durch eine erhöhte Stabilität gekennzeichnet sind.

Die Herstellung der erfindungsgemäßen Verbindungen erfolgt nach an sich bekannten Methoden wie beispielsweise in Houben-Weyl, "Methoden der organischen Chemie", Band 15 (1+2), Georg Thieme Verlag Stuttgart, 1974, von Erich Wünsch oder in Pharmazie 39, 228 (1984) beschrieben.

Vorzugsweise wird zunächst Boc-Cyanophenylalanin an die "Aminkomponente" gekoppelt. Als Aminkomponente werden bevorzugt cyclische Amine wie Piperidin, Methylpiperidin oder Hydroxymethylpiperidin eingesetzt. Die Herstellung der Peptidbindung erfolgt nach den gängigen Standardverfahren, wobei bevorzugt mit einem Carbodiimid in Gegenwart von Hydroxybenzotriazol in Dimethylformamid oder einem vergleichbaren Lösemittel gearbeitet wird.
Nach Isolation der N-alpha geschützten Verbindung wird die Schutzgruppe, in diesem Fall die Boc-Gruppe, mittels Acidolyse entfernt. Dazu wird vorzugsweise Trifluoressigsäure, gegebenenfalls in einem Lösemittel wie Dichlormethan, oder HCl in Essigsäure zur Abspaltung genutzt.

Die Ankopplung der Azaaminosäure erfolgt ebenfalls nach an sich bekannten Verfahren wie beispielsweise der Aktivestermethode, bei der ein geschütztes Hydrazinderivat mit Chlorameisensäureestern zu einem Aktivester umgesetzt wird. Als Aktivester werden besonders para-Nitrophenylester eingesetzt. Als weitere Möglichkeit kommt eine Aktivierung der Aminogruppe des Cyanophenylrestes zu einem Isocyanat mit nachfolgender Reaktion mit einem Hydrazinderivat in Frage. Die Nß-Funktionen der Hydrazine sind bei den Umsetzungen mit an sich bekannten Schutzgruppen blockiert, wozu besonders die Benzyloxycarbonylgruppe und ganz besonders die tert.-Butyloxycarbonylgruppe eingesetzt werden. Nach acidolytischer Abspaltung der Schutzgruppe wird ein aromatisches, heteroaromatisches oder heterocyclisches Sulfonsäurechlorid in einem Lösemittel wie Tetrahydrofuran, Dioxan, Dichlormethan oder DMF unter Zusatz einer Base wie N-Methylmorpholin, Triethylamin oder Diisopropylethylamin angekoppelt. Vielfach erweist es sich aber als günstig, zuerst das Sulfonsäurechloridderivat an das Hydrazinderivat zu koppeln. Damit kann die Einführung einer Schutzgruppe umgangen werden. Die Umsetzung der Cyano- in die Amidinofunktion erfolgt nach bekannten Reaktionsvorschriften. Vorzugsweise wird das entsprechende Cyanophenylalaninderivat in Triethylamin/Pyridin über einige Tage hinweg mit Schwefelwasserstoff behandelt. Die dadurch gebildeten Thioamide werden isoliert und mit einem Methylierungsmittel wie Methyliodid in die Thioimidsäuremethylesterverbindung überführt. Durch eine Behandlung mit Ammoniumverbindungen wie beispielsweise Ammoniumacetat, vorzugsweise in Methanol als Lösemittel, läßt sich die gewünschte Amidinophenylalaninverbindung erhalten.

Die Verbindungen werden nach gängigen Methoden gereinigt. Vorzugsweise wird die Gelpermeationschromatographie an Materialien wie ^{R}Sephadex LH-20 oder aber eine Ionenaustauscher-Chromatographie an Materialien wie CM-Cellulose benutzt, wobei besonders bevorzugt ein Acetatpuffer benutzt wird. Die erfindungsgemäßen Verbindungen werden mittels der Dünnschichtchromatographie und HPLC auf ihre Reinheit überprüft. Die Identität wird mittels Elementaranalyse und NMR geprüft.

Die erfindungsgemäßen Inhibitoren werden zur Beurteilung ihrer Wirksamkeit nach verschiedenen Kriterien geprüft, vorzugsweise sind dies die Bestimmung des Ki-Wertes, des IC50-Wertes oder die partielle Thromboplastinzeit (PTT) in vivo und in vitro. Demnach handelt es sich bei den beanspruchten Verbindungen um spezifische und hochaktive Thrombininhibitoren mit einem beträchtlichen antithrombotischen Potential, das die bisher bekannten niedermolekularen Inhibitoren deutlich übertrifft.

Gegenstand der Erfindung sind auch diagnostische Mittel oder therapeutische Mittel mit antithrombotischer Wirkung, welche die beschriebenen Inhibitoren enthalten, und die Verwendung dieser Verbindungen als Diagnostika oder in einem Verfahren zur Herstellung eines Arzneimittels mit antithrombotischer Wirkung.

Die folgenden Beispiele beschreiben die Erfindung näher:

### Beispiel 1

### Beta-Naphthylsulfonyl-azaglycyl-D-p-amidinophenylalanyl-piperidid

### 1. Boc-D-p-Cyanophenylalanyl-piperidid

50 g (255 mMol) p-Cyanobenzylbromid, 55 g (255 mMol) Acetamidomalonsäurediethylester und 2g Kaliumjodid wurden in 250 ml abs. Dioxan zum Sieden erhitzt. Hierzu wurden innerhalb von 3 Stunden eine frisch hergestellte Lösung von 6g (260 mMol) Natrium in Ethanol zugetropft. Nach weiteren 3 Stunden Rückflußkochen wurde auf 80 Grad abgekühlt und innerhalb von 3 Stunden 170 ml 3N Natronlauge zugesetzt. Der Ansatz wurde 4 Stunden auf 95 Grad erhitzt. Nach Abkühlung wird mit 6N HCl auf pH 1 eingestellt und das Dioxan abgedampft. Ein eventuell ausgefal lener Niederschlag wurde abfiltriert. Es wurde mit Natronlauge auf pH 9 eingestellt und 2 mal mit Ethylacetat extrahiert. Die wässrige Phase wurde nochmals mit Salzsäure auf pH 1 eingestellt, wobei N-Acetylcyanophenylalanin auskristallisierte. Die Kristalle wurden gesammelt, mehrmals mit Wasser gewaschen und im Hochvakuum getrocknet.
Ausbeute: 47 g (79,2 % d. Th.)
Reinheitsprüfung: DC Rf= 0.5 (Chloroform 50/Methanol 10/Eisessig 2.5//Volumenanteile)
24 g dieses Produktes wurden durch Zugabe von 3 N Natronlauge in 3 Liter Wasser gelöst und der pH wurde auf 6-6,5 eingestellt. Dazu wurden 500 mg Acylase zugesetzt und der Ansatz wurde 4 Tage bei 37 Grad inkubiert. Hierauf wurde die Lösung durch Ultrafiltration von der Acylase befreit und anschließend auf ein Volumen von 1 Liter eingeengt. Nach Einstellung auf pH 1 wurde mehrfach mit Ethylacetat extrahiert. Die organische Phase wurde mit wenig konz. Kochsalzlösung gewaschen, über Natriumsulfat getrocknet und das Lösemittel eingedampft. Man erhielt 8.2 g
N-Acetyl-D-cyanophenylalanin (82 % d. Th.).
Zu 8 g dieser Verbindung wurden 22 ml Eisessig und 4,3 ml konz. Salzsäure mit 40 ml Wasser gegeben und 24 Stunden zum Sieden erhitzt. Nach Abdampfen der Abspaltlösung und Nachschleppen anhaftender Säurespuren mit Methanol wurde aus Methanol/Diethylether umgefällt. Ausbeute: 6.6 g (85 % d. Th.)

5 g D-Cyanophenylalaninhydrochlorid wurden in 14 ml Wasser unter Zugabe von 7.5 ml Diisopropylethylamin gelöst. Dazu wurde eine Lösung von 6 g tert.-Butyloxycarbonyl-oxyimino-2-phenylacetonitril in 17 ml Dioxan zugesetzt und über Nacht gerührt. Es wurden 40 ml Wasser und 50 ml Ethylacetat zugesetzt. Die Wasserphase wurde abgetrennt und die organische Phase nochmals mit 1M Kaliumhydrogencarbonat extrahiert. Die vereinigten wässrigen Phasen wurden nochmals mit 10 ml Diethylether gewaschen und anschließend mit Salzsäure auf pH 3 eingestellt. Es wurde 3 mal mit Ethylacetat extrahiert, mit Natriumchloridlösung gewaschen und die organische Phase wurde über Natriumsulfat getrocknet. Nach Abdampfen des Lösemittels erhielt man 5.6 g (78 %) Boc-D-cyanophenylalanin.
3.26 g (10 mMol) Boc-D-Cyanophenylalanin, 1.49 g (11 mMol) HOBt und 2.42 g (12 mMol) DCCI wurden in 50 ml DMF gelöst und 1 Stunde gerührt. Es wurde 1 ml Piperidin zugesetzt und über Nacht gerührt. Ausgefallener Dicyclohexylharnstoff wurde abfiltriert, das DMF abdestilliert und der Rückstand in Ethylacetat aufgenommen. Es wurde 3 mal mit Kaliumhydrogencarbonat, 3 mal mit 1M Kaliumhydrogensulfat und 3 mal mit Ges. Kochsalzlösung gewaschen. Nach Trocknung der organischen Phase mit Natriumsulfat und Abdestillation des Lösemittels erhielt man 3.16 g (80 %) Boc-D-Cyanophenylalanyl-piperidid Reinheitskontrolle: DC Rf = 0.27 (Chloroform)

### 2. D-Cyanophenylalanyl-piperidin-hydrochlorid

3 g der Boc-geschützten Verbindung wurden in 50 ml 1.2 N HCl in Eisessig gelöst und 30 min bei Raumtemperatur gerührt. Das Abspaltreagens wurde im Vakuum abdestilliert, mit Toluol nachgeschleppt und der Rückstand mit wenig Diethylether verrieben. Die Kristalle wurden gesammelt und im Vakuum getrocknet.
Ausbeute: 2.2 g

### 3. Boc-Azaglycyl-D-cyanophenylalanyl-piperidid

2,08 g (7 mMol) Boc-Azaglycin-para-nitrophenylester und 2.06 g (7 mMol) Cyanophenylalanyl-piperidid wurden in 50 ml DMF gelöst. Nach Zusatz von 2.4 ml (14 mMol) Diisopropylethylamin wurde 1 Tag im Dunklen bei Raumtemperatur gerührt. Das Lösemittel wurde im Vakuum abdestilliert, der Rückstand in Ethylacetat aufgenommen und 3 mal mit 1M Kaliumhydrogensulfatlösung, 3 mal mit Kaliumhydrogencarbonatlösung und 2 mal mit konz. Kochsalzlösung gewaschen. Die organische Phase wurde über Natriumsulfat getrocknet und das Lösemittel abgedampft. Der Rückstand wurde mit Diisopropylether verrührt, die Kristalle gesammelt und getrocknet. Man erhielt 2.29 g Boc-Azaglycyl-D-cyanophenylalanyl-piperidid.

### 4. Beta-Naphthylsulfonyl-azaglycyl-D-cyanophenylalanyl-piperidid

2.08 g (5 mMol)
Boc-Azaglycyl-D-cyanophenylalanylpiperidid wurden in 50 ml 1.2 N HCl in Eisessig gelöst und 30 min bei Raumtempe ratur gerührt. Nach Abdampfen des Abspaltungsmittels und Nachschleppen mit Toluol im Vakuum wurde der Rückstand mit Ether verrieben und die Kristalle gesammelt. Die Kristalle wurden in 50 ml Dichlormethan unter Zusatz von 1.7 ml (10 mMol) Diisopropylethylamin gelöst. Hierzu wurden 1.134 g β-Naphthylsulfonylchlorid zugegeben und der Ansatz wurde über Nach bei Raumtemperatur gerührt. Das Lösemittel wurde abdestilliert, der Rückstand in Ethylacetat aufgenommen und 3 mal mit 1M Kaliumhydrogen-sulfatlösung, 3 mal mit Kaliumhydrogencarbonatlösung und 2 mal mit konz. Kochsalzlösung gewaschen. Die organische Phase wurde über Natriumsulfat getrocknet und das Lösemittel abgedampft. Man erhielt 1.75 g β-Naphthylsulfonyl-azaglycyl-D-cyanophenylalanyl-piperidid.

### 5. Beta-Naphthylsulfonyl-azaglycyl-D-amidino-phenylalanylpiperidid

1 g der in 4. erhaltenen Verbindung wurden in 30 ml trockenem Pyridin gelöst und nach Zugabe von 1 ml Triethylamin wurde 3 Stunden lang Schwefelwasserstoffgas eingeleitet. Nach dreitägigem Stehenlassen bei Raumtemperatur wurde auf eine Mischung aus 100 g Eis und 50 ml konz. Salzsäure gegossen. Der Niederschlag wurde abgesaugt und mit Wasser gewaschen. Das Thioamid wurde nach Trocknung in 50 ml Aceton aufgenommen und mit 1.5 ml Methyljodid versetzt. Es wurde 30 Minuten am Rückfluß gekocht. Nach Abkühlen wurde mit Diethylether gefällt. Der Niederschlag wurde in Dichlormethan gelöst und zweimal mit Wasser gewaschen. Nach Trocknung der organischen Phase über Natriumsulfat und Entfernung des Lösemittels wurde der Rückstand in 30 ml trockenem Methanol aufgenommen und 200 mg Ammoniumacetat zugegeben. Es wurde 3 Stunden auf 60 Grad erhitzt. Das Lösemittel wurde im Vakuum abgedampft. Das Produkt wurde einer chromatographischen Reinigung an ^{R}Sephadex LH-20 in Methanol unterzogen.
Ausbeute: 590 mg
Reinheitskontrolle: Schmelzpunkt 182°C
DC: RF = 0.48 (Chloroform 50/Methanol 10/ Eisessig 2.5 //Volumina)
Identitätskontrolle: Molmassenbestimmung (Fast atom bombardment) M⁺+H+ 523

### Beispiel 2

### Pmc-Azaglycyl-D-p-amidinophenylalanylpiperidid

Die Stufen 1.-3. sind mit dem vorhergehenden Beispiel identisch

### 4. Pmc-Azaglycyl-D-cyanophenylalaninpiperidid

1.67 g (4 mMol) Boc-Azaglycyl-D-cyanophenylalanylpiperidid wurden in 50 ml 1.2 N HCl in Eisessig gelöst und 30 min bei Raumtemperatur gerührt. Nach Abdampfen des Abspaltungsmittels und Nachschleppen mit Toluol im Vakuum wurde der Rückstand mit Ether verrieben und die Kristalle gesammelt. Die Kristalle wurden in 50 ml Dichlormethan unter Zusatz von 1.36 ml (8 mMol) Diisopropylethylamin gelöst. Hierzu wurden 1.35 g Pmc-chlorid zugegeben und der Ansatz wurde über Nacht bei Raumtemperatur gerührt. Das Lösemittel wurde abdestilliert, der Rückstand in Ethylacetat aufgenommen und 3 mal mit 1M Kaliumhydrogensulfatlösung, 3 mal mit Kaliumhydrogencarbonatlösung und 2 mal mit konz. Kochsalzlösung gewaschen. Die organische Phase wurde über Natriumsulfat getrocknet und das Lösemittel abgedampft. Man erhielt 1.95 g Pmc-Azaglycyl-D-cyanophenylalanylpiperidid.

### 5. Pmc-Azaglycyl-D-amidinophenylalanylpiperidid

1,5 g der in 4. erhaltenen Verbindung wurden in 30 ml trockenem Pyridin gelöst und nach Zugabe von 1 ml Triethylamin wurde 3 Stunden lang Schwefelwasserstoffgas eingeleitet. Nach dreitägigem Stehenlassen bei Raumtemperatur wurde auf eine Mischung aus 100 g Eis und 50 ml konz. Salzsäure gegossen. Der Niederschlag wurde abgesaugt und mit Wasser gewaschen. Das Thioamid wurde nach Trocknung in 50 ml Aceton aufgenommen und mit 1.5 ml Methyljodid versetzt. Es wurde 30 Minuten am Rückfluß gekocht. Nach Abkühlen wurde mit Diethylether gefällt. Der Niederschlag wurde in Dichlormethan gelöst und zweimal mit Wasser gewaschen Nach Trocknung der organischen Phase über Natriumsulfat und Entfernung des Lösemittels wurde der Rückstand in 30 ml trockenem Methanol aufgenommen und 300 mg Ammoniumacetat zugegeben. Es wurde 3 Stunden auf 60 Grad erhitzt. Das Lösemittel wurde im Vakuum abgedampft. Das Produkt wurde einer chromatographischen Reinigung an ^{R}Sephadex LH-20 in Methanol unterzogen. Ausbeute: 990 mg
Reinheitskontrolle: Schmelzpunkt 149-155°C (Sintern, Acetatsalz)
DC: RF = 0.52 (Chloroform 50/Methanol 10/ Eisessig 2.5 //Volumina)

### Bestimmung des IC50 Wertes der Thrombininhibition:

Die Verbindungen wurden in steigenden Konzentrationen mit humanem Thrombin in 0.1 M Tris-HCl-Puffer// 0.15 M NaCl pH 8.2 inkubiert. Nach einer Stunde wurde die enzymatische Reaktion durch die Zugabe des Substrates Chromozym^{R} TH (Tos-Gly-Pro-Arg-pNA, 5x10⁻⁵ M/l) gestartet. Die Freisetzung von pNA wurde nach einer Stunde als Zunahme der optischen Dichte bei 405 nm im Photometer gemessen. Die Konzentration an Inhibitor, die eine 50 % Hemmung der Enzymaktivität bewirkte, wurde als IC50 bezeichnet (100 % entspricht der nicht inhibierten Enzymreaktion).

### Bestimmung des KI-Wertes für Thrombin

Mit der o. g. Thrombinlösung wurden die KI-Werte für die untersuchten Verbindungen ermittelt. Dazu wurde Thrombin mit einer Konzentration an Inhibitor, der ungefähr dem im o. g. Test ermittelten IC₅₀-Wert entsprach, inkubiert. Die Reaktion wurde mit unterschiedlichen Konzentrationen des Substrates Chromozym TH (0.7- 45 x 10⁻⁵ Mol/l) gestartet. Der Hemmtyp und der KI-Wert wurde nach der von Lineweaver und Burk (J. Amer. Chem. Soc., 56, 658-666, 1934) beschriebenen Methode ermittelt.

**Tabelle 1**

| **Verbindung** | **Thrombinhemmung** | |
|---|---|---|
| | **IC50(mol/l)** | **KI(mol/l)** |
| Pmc-Gly-Aph-Pip | 1.4 x 10⁻⁹ | 1.3 x 10⁻⁹ |
| Nas-AGly-Aph-Pip | 1.3 x 10⁻⁹ | 2.6 x 10⁻⁹ |
| Pmc-AGly-Aph-Pip | 1.6 x 10⁻¹² | 9.2 x 10⁻¹¹ |
| NAPAP | 2.8 x 10⁻⁹ | 1.4 x 10⁻⁸ |

### Abkürzungen

- Boc: tert.-Butyloxycarbonyl
- DC: Dünnschichtchromatographie
- Rf: Retentionsfaktor
- HOBt: Hydroxybenzotriazol
- DCCI: Dicyclohexylcarbodiimid
- DMF: Dimethylformamid
- Pmc: 2,2,5,7,8-Pentamethylchroman-6-sulfonyl.

## Patentansprüche (Patentansprüche für folgende(n) Vertragsstaat(en): AT, BE, CH, DE, DK, FR, GB, IT, LI, LU, NL, PT, SE)

1. Verbindung der Struktur I 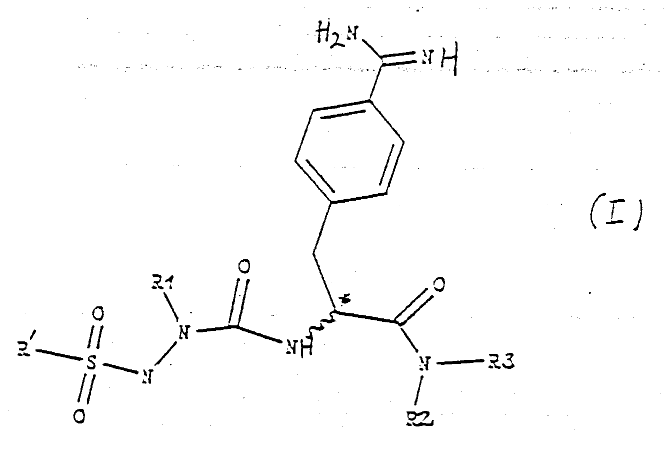 worin
R' eine Naphthyl- oder Phenylgruppe, die mit ein bis drei Alkoxygruppen, enthaltend bis zu drei C-Atome, oder mit bis zu fünf Alkylgruppen, enthaltend ein bis zwei C-Atome, derivatisiert sein kann, oder eine Chromangruppe, die mit bis zu fünf Methylgruppen derivatisiert sein kann,
R1 Wasserstoff, eine niedere Alkylgruppe mit bis zu 4 C-Atomen, eine Hydroxyalkylgruppe, eine Aralkylgruppe mit bis zu 7 Kohlenstoffatomen oder eine Carboxyalkylgruppe mit bis zu 4 C-Atomen,
R2 und R3 gleich oder verschieden je eine Alkylgruppe mit bis zu 4 C-Atomen bedeuten, wobei R² und R³ mit dem Stickstoffatom einen Ring bilden können, der mit einer Carboxyl-, Hydroxyl- oder Hydroxyalkylgruppe mit bis zu 3 Kohlenstoffatomen derivatisiert sein kann, und
* in der R oder S Struktur, vorzugsweise aber in der R-Struktur vorliegt.

2. Eine Verbindung nach Anspruch 1, in der R' β-Naphthyl, R1 Wasserstoff und R2 und R3 zusammen Piperidin sind.

3. Eine Verbindung nach Anspruch 1, in der R' 2,2,5,7,8-Pentamethylchroman, R1 Wasserstoff und R2 und R3 zusammen Piperidin sind.

4. Eine Verbindung nach Anspruch 1, in der R' β-Naphthyl, R1 -CH₂-COOH und R2 und R3 zusammen Piperidin sind.

5. Eine Verbindung nach Anspruch 1, in der R' β-Naphthyl, R1 Methyl und R2 und R3 zusammen Piperidin sind.

6. Eine Verbindung nach Anspruch 1, in der R' 6,7-Dimethoxy-β-naphthyl, R1 H und R2 und R3 zusammen Piperidin sind.

7. Eine Verbindung nach Anspruch 1, in der R' 5-Methoxy-alpha-naphthyl, R1 Methyl und R2 und R3 zusammen Piperidin sind.

8. Eine Verbindung nach Anspruch 1, in der R' β-Naphthyl, R1 H und R2 und R3 zusammen 3-Piperidin sind.

9. Eine Verbindung nach Anspruch 1, in der R' 5,6,7,8-Tetrahydro-β-naphthyl, R1 CH₂-COOH und R2 und R3 zusammen Piperidin sind.

10. Eine Verbindung nach Anspruch 1, in der R' Methoxytrimethylphenyl ist.

11. Eine Verbindung nach Anspruch 1, in der R' Pentamethylphenyl, R1 H und R2 und R3 zusammen Piperidin sind.

12. Verfahren zur Herstellung einer Verbindung der Formel (I), dadurch gekennzeichnet, daß ein Hydrazinderivat der Formel R'-SO₂-NH-NHR1, worin R' und R1 die in Anspruch 1 bezeichnete Bedeutung besitzen, mit einem üblichen Carbonylierungsmittel, bevorzugt Chlorameisensäure-para-nitrophenylester in Lösung umgesetzt wird und das Reaktionsprodukt mit para-Amidinophenyldialkylamid, worin die Alkylgruppen die in Anspruch 1 bezeichnete Bedeutung R2 und R3 haben, umgesetzt wird.

13. Diagnostisches oder therapeutisches Mittel enthaltend eine Verbindung nach Anspruch 1.

14. Verwendung einer Verbindung nach Anspruch 1 in einem Verfahren zur Herstellung eines Diagnostikums oder eines Arzneimittels mit antithrombotischer Wirkung.

## Patentansprüche (Patentansprüche für folgende(n) Vertragsstaat(en): ES, GR)

1. Verfahren zur Herstellung einer Verbindung der Formel (I), 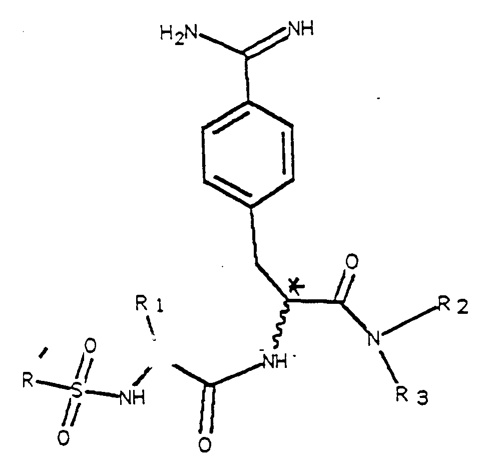 worin
R' eine Naphthyl- oder Phenylgruppe, die mit ein bis drei Alkoxygruppen, enthaltend bis zu drei C-Atome, oder mit bis zu fünf Alkylgruppen, enthaltend ein bis zwei C-Atome, derivatisiert sein kann, oder eine Chromangruppe, die mit bis zu fünf Methylgruppen derivatisiert sein kann,
R1 Wasserstoff, eine niedere Alkylgruppe mit bis zu 4 C-Atomen, eine Hydroxyalkylgruppe, eine Aralkylgruppe mit bis zu 7 Kohlenstoffatomen oder eine Carboxyalkylgruppe mit bis zu 4 C-Atomen,
R2 und R3 gleich oder verschieden je eine Alkylgruppe mit bis zu 4 C-Atomen bedeuten, wobei R² und R³ mit dem Stickstoffatom einen Ring bilden können, der mit einer Carboxyl-, Hydroxyl- oder Hydroryalkylgruppe mit bis zu 3 Kohlenstoffatomen derivatisiert sein kann, und
* in der R oder S Struktur, vorzugsweise aber in der R-Struktur vorliegt,
dadurch gekennzeichnet, daß ein Hydrazinderivat der Formel R'-SO₂-NH-NHR1, worin R' und R1 die zu Formel I angegebene Bedeutung besitzen, mit einem Carbonylierungsmittel in Lösung umgesetzt wird und das Reaktionsprodukt mit para-Amidinophenyldialkylamid, worin die Alkylgruppen die zu Foreml I für R2 und R3 Bedeutung haben, umgesetzt wird.

2. Verfahren nach Anspruch 1, dadurch gekennzeichnet, daß als Carbonylierungsmittel Chlorameisensäure-paranitrophenylester verwendet wird.

3. Verfahren nach Anspruch 1, worin eine Verbindung hergestellt wird, in der R' β-Naphthyl, R1 Wasserstoff und R2 und R3 zusammen Piperidin sind.

4. Verfahren nach Anspruch 1, in der R' 2,2,5,7,8-Pentamethylchroman, R1 Wasserstoff und R2 und R3
zusammen Piperidin sind.

5. Verfahren nach Anspruch 1, in der R' β-Naphthyl, R1 -CH₂-COOH und R2 und R3 zusammen Piperidin sind.

6. Verfahren nach Anspruch 1, in der R' β-Naphthyl, R1 Methyl und R2 und R3 zusammen Piperidin
sind.

7. Verfahren nach Anspruch 1, in der R' 6,7-Dimethoxy-β-naphthyl, R1 H und R2 und R3 zusammen Piperidin sind.

8. Verfahren nach Anspruch 1, in der R' 5-Methoxy-alpha-naphthyl, R1 Methyl und R2 und R3 zusammen Piperidin sind.

9. Verfahren nach Anspruch 1, in der R' β-Naphthyl, R1 H und R2 und R3 zusammen 3-Piperidin sind.

10. Verfahren nach Anspruch 1, in der R' 5,6,7,8-Tetrahydro-β-naphthyl, R1 CH₂-COOH und R2 und R3 zusammen Piperidin sind.

11. Verfahren nach Anspruch 1, in der R' Methoxytrimethylphenyl ist.

12. Verfahren nach Anspruch 1, in der R' Pentamethylphenyl, R1 H und R2 und R3 zusammen Piperidin sind.

## Claims (Claims for the following Contracting State(s): AT, BE, CH, DE, DK, FR, GB, IT, LI, LU, NL, PT, SE)

1. A compound of the structure I 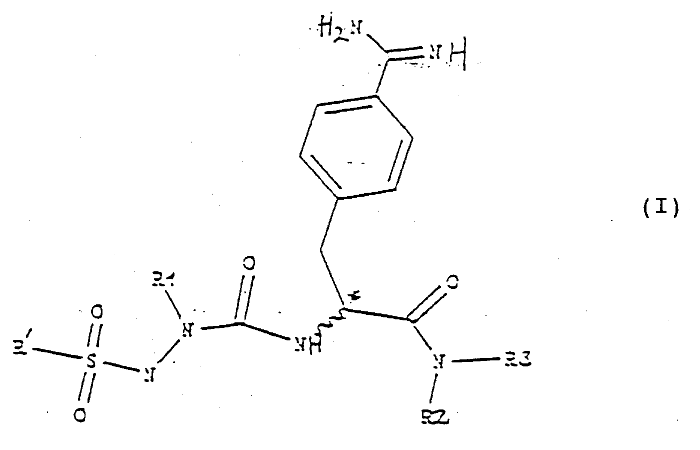 in which
R' is a naphthyl or phenyl group which can be derivatized with one to three alkoxy groups, containing up to three carbon atoms, or with up to five alkyl groups, containing one or two carbon atoms, or a chroman group which can be derivatized with up to five methyl groups,
R1 is hydrogen, a lower alkyl group having up to 4 carbon atoms, a hydroxyalkyl group, an aralkyl group having up to 7, carbon atoms or a carboxyalkyl group having up to 4 carbon atoms,
R2 and R3 are identical or different and in each case denote an alkyl group having up to 4 carbon atoms, where R2 and R3, together with the nitrogen atom, can form a ring which can be derivatized with a carboxyl group, hydroxyl group or hydroxyalkyl group having up to 3 carbon atoms, and
* exists in the R or S structure, but preferably in the R structure.

2. A compound as claimed in claim 1, in which R' is β-naphthyl, R1 is hydrogen and R2 and R3 are together piperidine.

3. A compound as claimed in claim 1, in which R' is 2,2,5,7,8-pentamethylchroman, R1 is hydrogen and R2 and R3 are together piperidine.

4. A compound as claimed in claim 1, in which R' is β-naphthyl, R1 is -CH₂-COOH and R2 and R3 are together piperidine.

5. A compound as claimed in claim 1, in which R' is β-naphthyl, R1 is methyl and R2 and R3 are together piperidine.

6. A compound as claimed in claim 1, in which R' is 6,7-dimethoxy-β-naphthyl, R1 is H and R2 and R3 are together piperidine.

7. A compound as claimed in claim 1, in which R' is 5-methoxy-alpha-naphthyl, R1 is methyl and R2 and R3 are together piperidine.

8. A compound as claimed in claim 1, in which R' is β-naphthyl, R1 is H and R2 and R3 are together 3-piperidine.

9. A compound as claimed in claim 1, in which R' is 5,6,7,8-tetrahydro-β-naphthyl, R1 is CH₂-COOH and R2 and R3 are together piperidine.

10. A compound as claimed in claim 1, in which R' is methoxytrimethylphenyl.

11. A compound as claimed in claim 1, in which R' is pentamethylphenyl, R1 is H and R2 and R3 are together piperidine.

12. A process for the preparation of a compound of the formula (I), which comprises reacting a hydrazine derivative of the formula R'-SO₂-NH-NHR1, in which R' and R1 have the meaning designated in claim 1, with a customary carbonylating agent, preferably para-nitrophenyl chloroformate, in solution and reacting the reaction product with para-amidinophenyldialkylamide, in which the alkyl groups have the meanings R2 and R3 designated in claim 1.

13. A diagnostic or therapeutic composition containing a compound as claimed in claim 1.

14. The use of a compound as claimed in claim 1 in a process for the preparation of a diagnostic or of a pharmaceutical having antithrombotic action.

## Claims (Claims for the following Contracting State(s): ES, GR)

1. A process for the preparation of a compound of the formula (I) 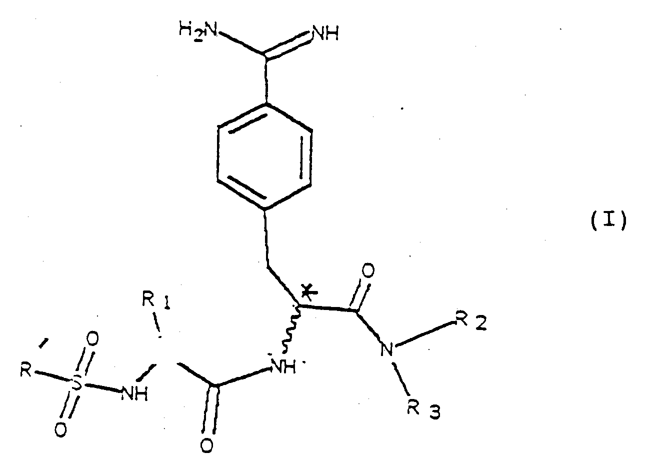 in which
R' is a naphthyl or phenyl group which can be derivatized with one to three alkoxy groups, containing up to three carbon atoms, or with up to five alkyl groups, containing one or two carbon atoms, or a chroman group which can be derivatized with up to five methyl groups,
R1 is hydrogen, a lower alkyl group having up to 4 carbon atoms, a hydroxyalkyl group, an aralkyl group having up to 7 carbon atoms or a carboxyalkyl group having up to 4 carbon atoms,
R2 and R3 are identical or different and in each case denote an alkyl group having up to 4 carbon atoms, where R2 and R3, together with the nitrogen atom, can form a ring which can be derivatized with a carboxyl group, hydroxyl group or hydroxyalkyl group having up to 3 carbon atoms, and
* exists in the R or S structure, but preferably in the R structure, which comprises reacting a hydrazine derivative of the formula R'-SO₂-NH-NHR1, in which R' and R1 have the meaning given in formula I, with a carbonylating agent in solution and reacting the reaction product with para-amidinophenyldialkylamide, in which the alkyl groups have the meaning given in formula I for R2 and R3.

2. The process as claimed in claim 1, wherein the carbonylating agent used is para-nitrophenyl chloroformate.

3. The process as claimed in claim 1, in which a compound is prepared in which R' is β-naphthyl, R1 is hydrogen and R2 and R3 are together piperidine.

4. The process as claimed in claim 1, in which R' is 2,2,5,7,8-pentamethylchroman, R1 is hydrogen and R2 and R3 are together piperidine.

5. The process as claimed in claim 1, in which R' is β-naphthyl, R1 is -CH₂-COOH and R2 and R3 are together piperidine.

6. The process as claimed in claim 1, in which R' is β-naphthyl, R1 is methyl and R2 and R3 are together piperidine.

7. The process as claimed in claim 1, in which R' is 6,7-dimethoxy-β-naphthyl, R1 is H and R2 and R3 are together piperidine.

8. The process as claimed in claim 1, in which R' is 5-methoxy-alpha-naphthyl, R1 is methyl and R2 and R3 are together piperidine.

9. The process as claimed in claim 1, in which R' is β-naphthyl, R1 is H and R2 and R3 are together 3-piperidine.

10. The process as claimed in claim 1, in which R' is 5,6,7,8-tetrahydro-β-naphthyl, R1 is CH₂-COOH and R2 and R3 are together piperidine.

11. The process as claimed in claim 1, in which R' is methoxytrimethylphenyl.

12. The process as claimed in claim 1, in which R' is pentamethylphenyl, R1 is H and R2 and R3 are together piperidine.

## Revendications (Revendications pour l'(les) Etat(s) contractant(s) suivant(s): AT, BE, CH, DE, DK, FR, GB, IT, LI, LU, NL, PT, SE)

1. Composé de formule I 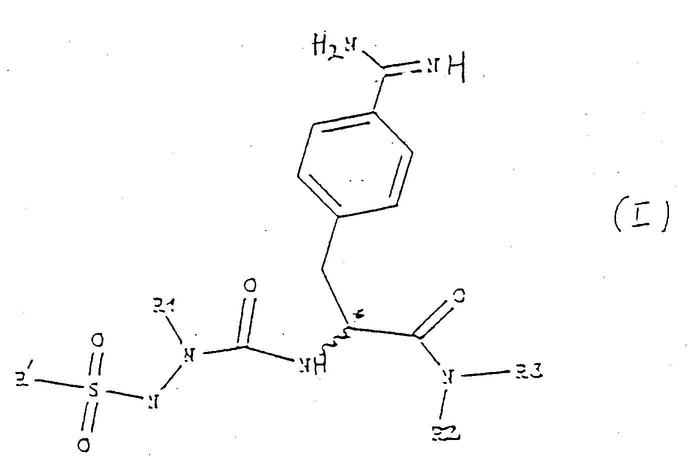 dans laquelle
R' représente un radical naphtyle ou phényle qui peut être substitué par 1 à 3 groupes alcoxy contenant jusqu'à trois atomes de carbone ou par jusqu'à 5 groupes alkyle contenant 1 ou 2 atomes de carbone, ou un radical chromane qui peut être substitué par jusqu'à 5 groupes méthyle,
R¹ représente un atome d'hydrogène ou un groupe alkyle inférieur ayant jusqu'à 4 atomes de carbone, un groupe hydroxyalkyle, un groupe aralkyle ayant jusqu'à 7 atomes de carbone ou un groupe carboxyalkyle ayant jusqu'à 4 atomes de carbone,
R² et R³ sont identiques ou différents et représentent chacun un groupe alkyle ayant jusqu'à 4 atomes de carbone, R² et R³ pouvant former avec l'atome d'azote un cycle qui peut être substitué par un groupe carboxy, hydroxy ou hydroxyalkyle ayant jusqu'à 3 atomes de carbone, et
* se trouve en configuration R ou S, mais de préférence en configuration R.

2. Composé selon la revendication 1, dans lequel R' est le groupe β-naphtyle, R¹ représente un atome d'hydrogène et R² et R³ forment ensemble le cycle pipéridine.

3. Composé selon la revendication 1, dans lequel R' est le groupe 2,2,5,7,8-pentaméthylchromane, R¹ est un atome d'hydrogène, et R² et R³ forment ensemble le cycle pipéridine.

4. Composé selon la revendication 1, dans lequel R' est le groupe β-naphtyle, R¹ est -CH₂-COOH, et R² et R³ forment ensemble le cycle pipéridine.

5. Composé selon la revendication 1, dans lequel R' est le groupe β-naphtyle, R¹ est le groupe méthyle, et R² et R³ forment ensemble le cycle pipéridine.

6. Composé selon la revendication 1, dans lequel R' est le groupe 6,7-diméthoxy-β-naphtyle, R¹ est H, et R² et R³ forment ensemble le cycle pipéridine.

7. Composé selon la revendication 1, dans lequel R' est le groupe 5-méthoxy-α-naphtyle, R¹ est le groupe méthyle, et R² et R³ forment ensemble le cycle pipéridine.

8. Composé selon la revendication 1, dans lequel R' est le groupe β-naphtyle, R¹ est H, et R² et R³ forment ensemble le cycle 3-pipéridine.

9. Composé selon la revendication 1, dans lequel R' est le groupe 5,6,7,8-tétrahydro-β-naphtyle, R¹ est CH₂-COOH, et R² et R³ forment ensemble le cycle pipéridine.

10. Composé selon la revendication 1, dans lequel R' est le groupe méthoxytriméthylphényle.

11. Composé selon la revendication 1, dans lequel R' est le groupe pentaméthylphényle, R¹ est H, et R² et R³ forment ensemble le cycle pipéridine.

12. Procédé pour la préparation d'un composé de formule (I), caractérisé en ce que l'on fait réagir en solution un dérivé d'hydrazine de formule R'-SO₂-NH-NHR¹, dans laquelle R' et R¹ont les significations indiquées dans la revendication 1, avec un agent de carbonylation usuel, de préférence le chloroformiate de p-nitrophényle, et on fait réagir le produit de réaction avec un p-amidinophényldialkylamide dans lequel les groupes alkyle ont la signification donnée pour R² et R³ dans la revendication 1.

13. Agent thérapeutique ou de diagnostic, contenant un composé selon la revendication 1.

14. Utilisation d'un composé selon la revendication 1, dans un procédé pour la préparation d'un agent de diagnostic ou d'un médicament à activité antithrombotique.

## Revendications (Revendications pour l'(les) Etat(s) contractant(s) suivant(s): ES, GR)

1. Procédé pour la préparation d'un composé de formule (I) 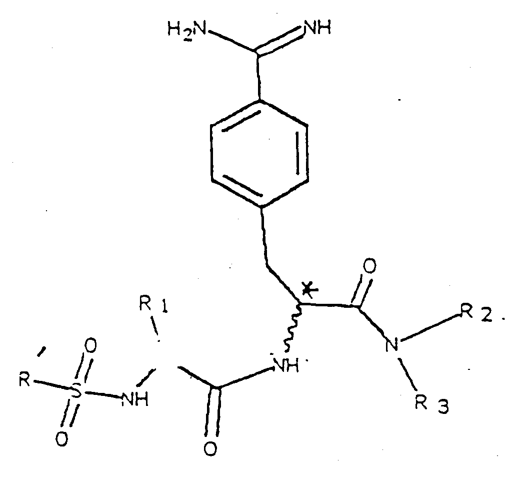 dans laquelle
R' représente un radical naphtyle ou phényle qui peut être substitué par 1 à 3 groupes alcoxy contenant jusqu'à trois atomes de carbone ou par jusqu'à 5 groupes alkyle contenant 1 ou 2 atomes de carbone, ou un radical chromane qui peut être substitué par jusqu'à 5 groupes méthyle,
R¹ représente un atome d'hydrogène ou un groupe alkyle inférieur ayant jusqu'à 4 atomes de carbone, un groupe hydroxyalkyle, un groupe aralkyle ayant jusqu'à 7 atomes de carbone ou un groupe carboxyalkyle ayant jusqu'à 4 atomes de carbone,
R² et R³ sont identiques ou différents et représentent chacun un groupe alkyle ayant jusqu'à 4 atomes de carbone, R² et R³ pouvant former avec l'atome d'azote un cycle qui peut être substitué par un groupe carboxy, hydroxy ou hydroxyalkyle ayant jusqu'à 3 atomes de carbone, et
* se trouve en configuration R ou S, mais de préférence en configuration R,
caractérisé en ce que l'on fait réagir en solution un dérivé d'hydrazine de formule R'-SO₂-NH-NHR¹, dans laquelle R' et R¹ont les significations indiquées à propos de la formule I, avec un agent de carbonylation, et on fait réagir le produit de réaction avec un p-amidinophényldialkylamide dans lequel les groupes alkyle ont la signification donnée pour R² et R³ à propos de la formule I.

2. Procédé selon la revendication 1, caractérisé en ce que, comme agent de carbonylation, on utilise le chloroformiate de p-nitrophényle.

3. Procédé selon la revendication 1, dans lequel on prépare un composé dans lequel R' est le groupe β-naphtyle, R¹est un atome d'hydrogène et R² et R³ forment ensemble le cycle pipéridine.

4. Procédé selon la revendication 1, dans lequel R' est le groupe 2,2,5,7,8-pentaméthylchromane, R¹ est un atome d'hydrogène, et R² et R³ forment ensemble le cycle pipéridine.

5. Procédé selon la revendication 1, dans lequel R' est le groupe β-naphtyle, R¹ est -CH₂-COOH, et R² et R³ forment ensemble le cycle pipéridine.

6. Procédé selon la revendication 1, dans lequel R' est le groupe β-naphtyle, R¹ est le groupe méthyle, et R² et R³ forment ensemble le cycle pipéridine.

7. Procédé selon la revendication 1, dans lequel R' est le groupe 6,7-diméthoxy-β-naphtyle, R¹ est H, et R² et R³ forment ensemble le cycle pipéridine.

8. Procédé selon la revendication 1, dans lequel R' est le groupe 5-méthoxy-α-naphtyle, R¹ est le groupe méthyle, et R² et R³ forment ensemble le cycle pipéridine.

9. Procédé selon la revendication 1, dans lequel R' est le groupe β-naphtyle, R¹ est H, et R² et R³ forment ensemble le cycle 3-pipéridine.

10. Procédé selon la revendication 1, dans lequel R' est le groupe 5,6,7,8-tétrahydro-β-naphtyle, R¹ est CH₂-COOH, et R² et R³ forment ensemble le cycle pipéridine.

11. Procédé selon la revendication 1, dans lequel R' est le groupe méthoxytriméthylphényle.

12. Procédé selon la revendication 1, dans lequel R' est le groupe pentaméthylphényle, R¹ est H, et R² et R³ forment ensemble le cycle pipéridine.
